# EUROPEAN PATENT APPLICATION

(11) **EP 2 987 454 A1**
(43) Date of publication of application: **24.02.2016**
(21) Application number: 14785018.4
(22) Date of filing: 16.04.2014
(51) Int. Cl.: A61B 6/03

(54) **X-RAY CT DEVICE**

(30) Priority: 16.04.2013 JP 2013085965
(71) Applicant: Kabushiki Kaisha Toshiba, Minato-ku Tokyo 105-8001 (JP); Toshiba Medical Systems Corporation, Otawara-shi, Tochigi 324-8550 (JP)
(72) Inventor: TAMURA, Emi, Otawara-shi Tochigi 324-8550 (JP); SAITO, Yasuo, Otawara-shi Tochigi 324-8550 (JP); TAKAYAMA, Takuzo, Otawara-shi Tochigi 324-8550 (JP); MIYAZAKI, Hiroaki, Otawara-shi Tochigi 324-8550 (JP); NAKAI, Hiroaki, Otawara-shi Tochigi 324-8550 (JP)
(74) Representative: Moreland, David
(86) International application number: PCT/JP2014/060830
(87) International publication number: WO 2014/171487

(57) **Abstract**

The energy crosstalk between X-rays generated by different tube voltages is reduced. An X-ray CT apparatus includes an X-ray tube (5), a high voltage generation unit (10) configured to selectively generate first and second tube voltages, a first filter (61a) formed from a material having substantially the same atomic number as that of a contrast material and configured to perform radiation quality adjustment, a second filter (61b) formed from a material different from the contrast material and configured to perform radiation quality adjustment, a filter switching mechanism (62) configured to switch between the first filter and the second filter, an X-ray detector (8), a reconstruction unit (42) configured to reconstruct an image based on projection data obtained by an output from the X-ray detector, and a control unit (12) configured to control the high voltage generation unit and the filter switching mechanism to synchronize switching between the first and second tube voltages with switching between the first and second filters.

## Description

### Technical Field

Embodiments described herein relate generally to an X-ray CT (Computed Tomography) apparatus.

### Background Art

There is available an X-ray CT apparatus which performs dual energy scanning. In dual energy scanning, the X-ray CT apparatus acquires projection data by using X-rays having a spectrum distribution in a low energy region (to be referred to as low-energy X-rays hereinafter) and also acquires projection data by using X-rays having a spectrum distribution in a high energy region (to be referred to as high-energy X-rays hereinafter) during scanning on an object. Using the projection data acquired in this manner can perform imaging using the differences in X-ray absorption coefficient between substances constituting an object. This makes it possible to obtain an image with an area contrast-enhanced by, for example, a contrast medium being separated from the remaining areas with a high contrast.

As the system configurations of X-ray CT apparatuses which perform dual energy scanning, there are known, for example, a dual-tube CT system, a fast switching CT system, and a dual-layer CT system.

A dual-tube CT system is a system which is equipped with two sets of X-ray tubes and X-ray detectors and is designed to acquire projection data using both low-energy X-rays corresponding to a low tube voltage and high-energy X-rays corresponding to a high tube voltage by one rotation by setting the tube voltages of the respective X-ray tubes to the low tube voltage and the high tube voltage, respectively.

A fast switching CT system is a system designed to acquire projection data using both low-energy X-rays and high-energy X-rays by one rotation by switching the tube voltage to be applied to the X-ray tube between a low tube voltage and a high tube voltage at high speed.

A dual-layer CT system is a system which includes X-ray detectors having a dual-layer structure and is designed to simultaneously acquire projection data corresponding to two energies by making the upper detector detect the low-energy portion of the X-rays generated by the X-ray tube and making the lower detector detect the high-energy portion.

Note that X-ray CT apparatuses using these systems are also called spectral CT.

Conventional spectral CT has a problem that the contrast of an image is degraded by the overlapping between the spectrum of low-energy X-rays and the spectrum of high-energy X-rays described above, i.e., so-called energy crosstalk.

### Citation List

### Patent Literature

Patent Literature 1: Jpn. Pat. Appln. KOKAI Publication No. 2012-245142

### Summary of Invention

### Technical Problem

The problem to be solved by the present invention is to reduce the above energy crosstalk.

### Solution to Problem

An X-ray CT apparatus according to this embodiment comprises an X-ray tube, a high voltage generation unit configured to selectively generate first and second tube voltages, a first filter formed from a material having substantially the same atomic number as that of a contrast material and configured to adjust radiation quality, a second filter formed from a material different from the contrast material and configured to adjust radiation quality, a filter switching mechanism configured to switch between the first and second filters, an X-ray detector, a reconstruction unit configured to reconstruct an image based on projection data output from the X-ray detector, and a control unit configured to control the high voltage generation unit and the filter switching mechanism to synchronize switching between the first and second tube voltages with switching between the first and second filters.

### Brief Description of Drawings

[FIG. 1] FIG. 1 is a block diagram showing an arrangement indicating the main part of an X-ray CT apparatus according to an embodiment.
[FIG. 2] FIG. 2 is a graph for explaining energy crosstalk.
[FIG. 3] FIG. 3 is a perspective view showing the schematic arrangement of a filter unit according to this embodiment.
[FIG. 4] FIG. 4 is a graph showing a spectrum Sa of X-rays emerging from an X-ray filter 61a and a spectrum Sb of X-rays emerging from an X-ray filter 61b when iodine is a specific material according to this embodiment.
[FIG. 5] FIG. 5 is a graph showing the spectrum Sa of X-rays emerging from the X-ray filter 61a and the spectrum Sb of X-rays emerging from the X-ray filter 61b when gadolinium is a specific material according to this embodiment.
[FIG. 6] FIG. 6 is a view for explaining a modification of this embodiment.
[FIG. 7] FIG. 7 is a perspective view showing another arrangement example of the filter unit according to this embodiment.
[FIG. 8] FIG. 8 is a chart showing a slow switching scheme for tube voltages and X-ray filters according to this embodiment.
[FIG. 9] FIG. 9 is a chart showing a fast switching scheme for tube voltages and X-ray filters according to this embodiment.
[FIG. 10] FIG. 10 is a perspective view showing the switching of a single X-ray filter according to this embodiment.
[FIG. 11] FIG. 11 is a graph showing the spectrum Sa of X-rays emerging from the X-ray filter 61a and a spectrum Sb' of X-rays directly emerging without using the X-ray filter 61b when indium and zinc are specific materials according to this embodiment.
[FIG. 12] FIG. 12 is a graph showing a spectrum Sa' of X-rays directly emerging without using the X-ray filter 61a and the spectrum Sb of X-rays emerging from the X-ray filter 61b when indium and zinc are specific materials according to this embodiment.
[FIG. 13] FIG. 13 is a graph showing the spectrum Sa' in FIG. 12 upon scaling down along the ordinate.

### Description of Embodiments

An embodiment will be described with reference to the accompanying drawings.

This embodiment discloses an X-ray CT apparatus, as an example of a spectral CT system, which switches the tube voltage to be applied to an X-ray tube between a low tube voltage and a high tube voltage every time the X-ray tube makes one rotation.

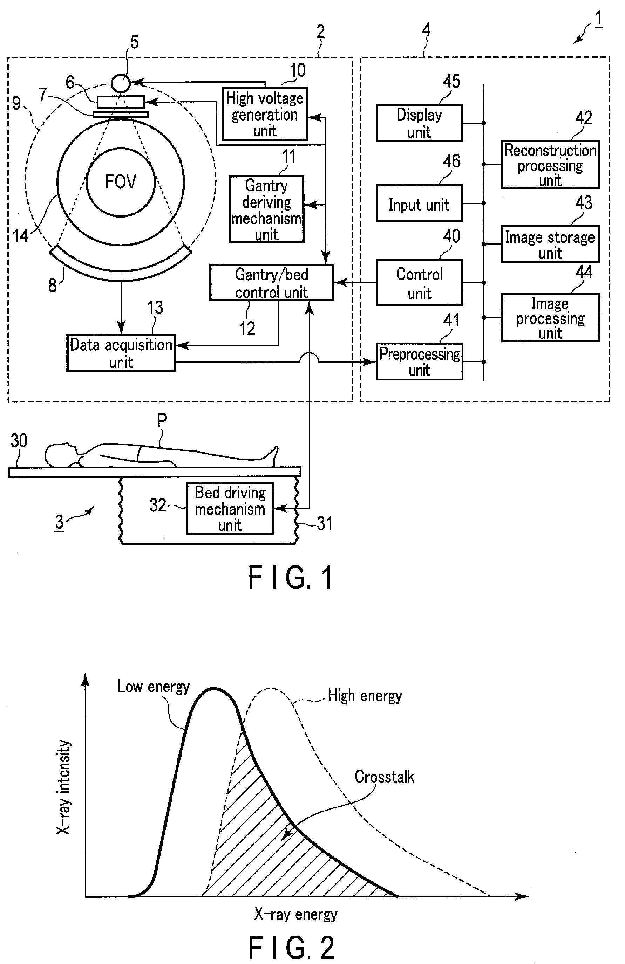

FIG. 1 is a block diagram showing an arrangement indicating the main part of an X-ray CT apparatus 1 according to this embodiment. As shown in FIG. 1, the X-ray CT apparatus 1 includes a gantry device 2, a bed device 3, and a console device 4.

The gantry device 2 includes an X-ray tube 5, a filter unit 6, an X-ray stop unit 7, an X-ray detector 8, a rotating frame 9, a high voltage generation unit 10, a gantry deriving mechanism unit 11, a gantry/bed control unit 12, and a data acquisition unit 13. In addition, the gantry device 2 includes an opening portion 14 as an imaging space into which an object P is sent.

The X-ray tube 5, the filter unit 6, the X-ray stop unit 7, and the X-ray detector 8 are mounted on the rotating frame 9. The gantry deriving mechanism unit 11 is constituted by a structural mechanism which rotates the rotating frame 9, a motor which operates the mechanism, and the like. As the rotating frame 9 rotates, the X-ray tube 5 and the X-ray detector 8 rotate around the object P transferred into the opening portion 14 while facing each other.

The high voltage generation unit 10 generates a filament current and a tube voltage. The filament current is supplied to the cathode filament of the X-ray tube 5. The tube voltage is applied between the two electrodes of the X-ray tube 5. The thermoelectrons generated by the cathode filament collide with the anode. This generates X-rays. The high voltage generation unit 10 can selectively generate a first tube voltage Va and a second tube voltage Vb higher than the first tube voltage Va under the control of the gantry/bed control unit 12.

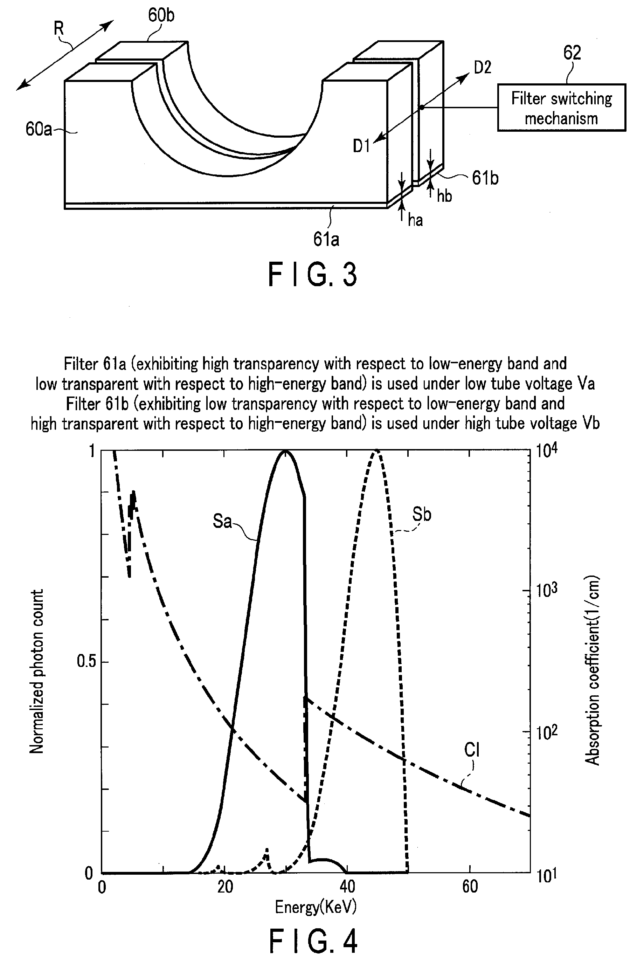

As exemplarily shown in FIG. 3, the filter unit 6 includes two wedge filters 60a and 60b having the same shape and material and two different types of X-ray filters 61a and 61b having different radiation quality adjustment characteristics. The filter 61a (first filter) is attached to the bottom surface of the wedge filter 60a. The filter (second filter) 61b is attached to the bottom surface of the wedge filter 60b.

The wedge filters 60a and 60b adjust the intensity of X-rays generated by the X-ray tube 5 so as to decrease the intensity from the scan center to the outside. The X-ray filters 61a and 61b further adjust the radiation quality of X-rays transmitted through the wedge filters. The X-ray filter (first filter) 61a is formed from a material having substantially the same atomic number as that of the contrast material administered to the object. The X-ray filter (second filter) 61b is formed from a material different from the contrast material and the first filter 61a. Typically, the second filter 61b is formed from a material having a higher atomic number than the contrast material and the first filter 61a. However, this description does not deny the possibility that the second filter 61b may be formed from a material having a lower atomic number than the contrast material and the first filter 61a.

The wedge filters 60a and 60b are arranged side by side along a rotation axis R. The filter unit 6 includes a filter switching mechanism 62. The filter switching mechanism 62 has a structure and motive power required to reciprocate the wedge filters 60a and 60b in a direction parallel to the rotation axis R. The filter switching mechanism 62 reciprocates the wedge filters 60a and 60b to switch them between the X-ray tube 5 and the object. This also switches the X-ray filters 61a and 61b between the X-ray tube 5 and the object.

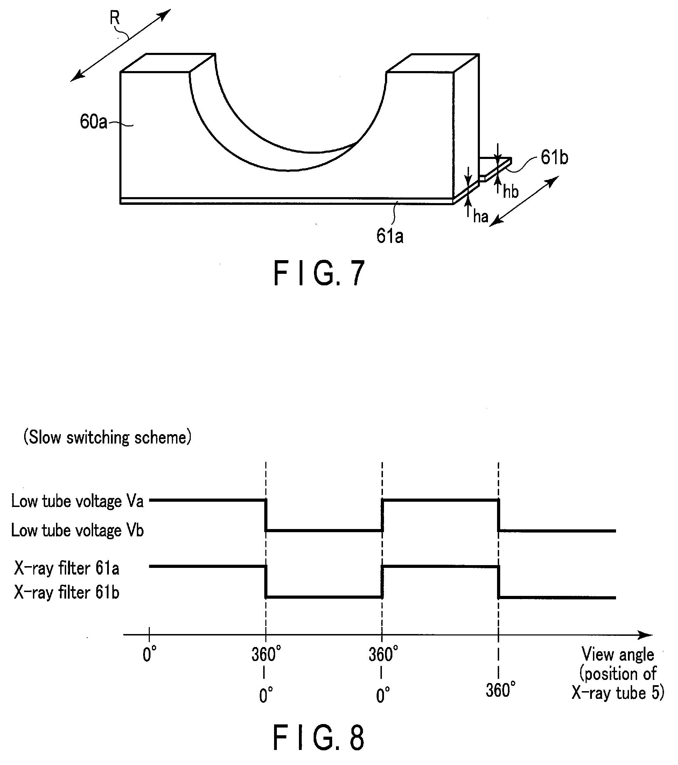

Note that, as shown in FIG. 7, the filter unit 6 may include the single wedge filter 60a. The single wedge filter 60a is fixed on an X-ray beam. The array of the X-ray filters 61a and 61b is separated from the wedge filter 60a. The filter switching mechanism 62 reciprocates the array of the X-ray filters 61a and 61b.

The X-ray stop unit 7 includes a plurality of slit plates. The plurality of slit plates each are movably supported. The plurality of slit plates each are moved to arbitrarily adjust the irradiation range of X-rays with which the object P are irradiated.

The X-ray detector 8 is a two-dimensional array detector (a so-called multi-slice detector), and includes a plurality of X-ray detection elements arrayed two-dimensionally.

The data acquisition unit (DAS) 13 receives the electrical signal output from each X-ray detection element of the X-ray detector 8, amplifies the received electrical signal, and converts the amplified electrical signal into a digital signal. The digital signal after the conversion is called projection data.

The bed device 3 includes a top 30 on which the object P is placed, a top support unit 31 which supports the top 30, and a bed driving mechanism unit 32.

The bed driving mechanism unit 32 is constituted by a structural mechanism which moves the top 30 in directions parallel and vertical to its mount surface, a motor which operates the mechanism, and the like. When performing scanning, the bed driving mechanism unit 32 transfers the top 30 into the opening portion 14 under the control of the gantry/bed control unit 12, thereby positioning the object P in the imaging area (FOV) of the gantry device 2.

The gantry/bed control unit 12 is constituted by a CPU (Central Processing Unit), a ROM (Read Only Memory), a RAM (Random Access Memory), and the like, and controls the respective units of the gantry device 2 and the bed device 3 in accordance with instructions input from a control unit 40 or the like of the console device 4.

The gantry/bed control unit 12 controls the high voltage generation unit 10 and the filter switching mechanism 62 to synchronize switching between the first and second tube voltages with switching between the first and second X-ray filters 61a and 61b. The first filter 61a is selected to select the first tube voltage. The second filter 61b is selected to select the second tube voltage. In this embodiment, operation schemes include the slow switching scheme and the fast switching scheme. It is possible to select the slow switching scheme or the fast switching scheme in accordance with an operator instruction.

FIG. 8 is a timing chart showing the operation of the slow switching scheme. The control unit 12 performs control to alternately switch between a state in which the low tube voltage Va is selected and the first filter 61a is arranged on an X-ray beam and a state in which the high tube voltage Vb is selected and the second filter 61b is arranged on an X-ray beam, every time the X-ray tube 5 rotates through an angle range necessary for image reconstruction, 360° in this case. An angle range necessary for image reconstruction is given by (180° + α), where α is the fan angle of X-rays, according to a so-called half reconstruction method.

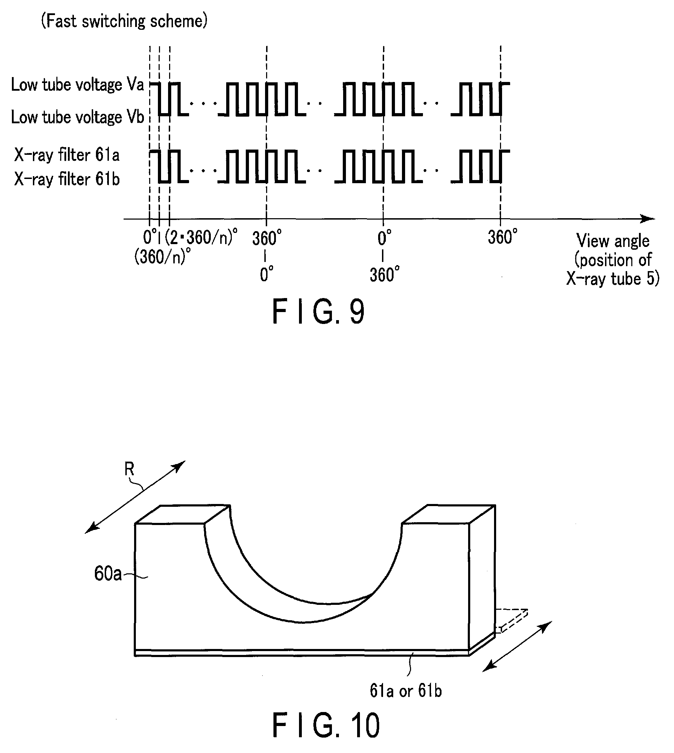

FIG. 9 is a timing chart showing the operation of the fast switching scheme. The control unit 12 performs control to alternately switch between a state in which the low tube voltage Va is selected and the first filter 61a is arranged and a state in which the high tube voltage Vb is selected and the second filter 61b is arranged, every time the X-ray tube 5 rotates through a view pitch (360°/n, where n is the number of samplings per rotation) or its integer multiple.

The console device 4 includes the control unit 40, a preprocessing unit 41, a reconstruction processing unit 42, an image storage unit 43, an image processing unit 44, a display unit 45, and an input unit 46.

The control unit 40 is constituted by a CPU, a ROM, a RAM, and the like, and controls the respective units of the console device 4.

The preprocessing unit 41 receives projection data from the data acquisition unit 13, and performs preprocessing such as sensitivity correction and X-ray intensity correction.

The reconstruction processing unit 42 generates reconstruction image data such as the tomographic image data or volume data of an object by reconstructing the projection data having undergone preprocessing by the preprocessing unit 41 in accordance with parameters such as a reconstruction slice thickness, reconstruction interval, and reconstruction function and a reconstruction protocol. The reconstruction function is a function for changing the contrast resolution and spatial resolution in accordance with the organ to be imaged and an examination purpose. The reconstruction protocol is defined by, for example, the type of algorithm used for reconstruction.

The image storage unit 43 stores the projection data (raw data) sent from the data acquisition unit 13, the projection data having undergone preprocessing by the preprocessing unit 41, the reconstruction image data generated by the reconstruction processing unit 42, and the like.

The image processing unit 44 performs image processing for display such as window conversion and RGB processing for the reconstruction image data stored in the image storage unit 43, and outputs the data after the processing to the display unit 45. The image processing unit 44 sometimes generates the data of a tomographic image of an arbitrary slice, a projection image from an arbitrary direction, a three-dimensional surface image, or the like by using the reconstruction image data based on an operator instruction, and outputs the data to the display unit 45. The display unit 45 displays the X-ray CT image based on the data output from the image processing unit 44.

The image processing unit 44 generates a desired image with improved contrast and an enhanced contrast material by performing weighted addition of the image reconstructed based on the projection data acquired while the first tube voltage Va is selected and the first filter 61a is arranged and the image reconstructed based on the projection data acquired while the second tube voltage Vb is selected and the second filter 61b is arranged.

The input unit 46 includes devices such as a keyboard, various types of switches, a mouse, and a trackball. The input unit 46 is used to input various types of scan conditions such as a scan protocol and a reconstruction protocol.

The X-ray CT apparatus 1 has a function of executing dual energy scanning. When executing dual energy scanning, the gantry/bed control unit 12 executes the first and second scans while switching them every time the X-ray tube 5 makes one rotation. The first scan is the processing of acquiring projection data by generating X-rays by applying a low tube voltage to the X-ray tube 5. The second scan is the processing of acquiring projection data by generating X-rays by applying a high tube voltage to the X-ray tube 5. In the following description, the low tube voltage Va is applied to the X-ray tube 5 in the first scan, and the high tube voltage Vb (Va < Vb) is applied to the X-ray tube 5 in the second scan.

The control unit 40 generates image data in accordance with the purpose of radiographic interpretation by performing weighted addition, at an arbitrary ratio, with respect to the reconstruction image data generated by the reconstruction processing unit 42 based on the projection data acquired by the first scan and the reconstruction image data generated by the reconstruction processing unit 42 based on the projection data acquired by the second scan. For example, the control unit 40 can generate image data with only a contrast-enhanced area of the object P being extracted.

A general problem caused in dual energy scanning will be described below with reference to FIG. 2. FIG. 2 is a graph showing the spectrum of X-rays generated upon application of a low tube voltage (e.g., 40 kV) to the X-ray tube and the spectrum of X-rays generated upon application of a high tube voltage (e.g., 50 kV) to the X-ray tube. A conventional spectral CT system has a problem that these two spectra partly overlap to cause so-called energy crosstalk.

This embodiment is configured to optimize the tube voltages to be applied to the X-ray tube 5 and the above X-ray filters so as to reduce or remove this energy crosstalk.

The details of the filter unit 6 will be described below. FIG. 3 is a perspective view showing the schematic arrangement of the filter unit 6. The filter unit 6 includes the wedge filters 60a and 60b, the X-ray filters 61a and 61b, and the filter switching mechanism 62. An arrow R shown in FIG. 3 indicates the rotation axis direction of the X-ray tube 5.

The wedge filters 60a and 60b have the same shape and are arranged side by side in the rotation axis direction. The upper surfaces of the wedge filters 60a and 60b are incident surfaces which X-rays from the X-ray tube 5 strike, and the lower surfaces of them are exit surfaces from which X-rays emerge. Each incident surface is a curved surface which is recessed toward the exist surface to have a shape in consideration of the body thickness of the object P. In contrast to this, each exit surface is a flat surface. Using the wedge filters 60a and 60b, each having such shape, can increase the intensity of X-rays with which a portion of the object P which has a large body thickness is irradiated and decrease the intensity of X-rays with which a portion of the object P which has a small body thickness is irradiated.

The X-ray filter 61a is a flat plate having a uniform thickness ha, and is fixed to the lower surface of the wedge filter 60a. The X-ray filter 61b is a flat plate having a uniform thickness hb, and is fixed to the lower surface of the wedge filter 60b.

The gantry/bed control unit 12 controls the filter switching mechanism 62. The filter switching mechanism 62 shifts the wedge filters 60a and 60b in directions D1 and D2 parallel to the above rotation axis direction. With this shifting operation, the gantry/bed control unit 12 can switch the X-ray filter to be interposed between the X-ray tube 5 and the object P between the X-ray filters 61a and 61b.

In this embodiment, in particular, the gantry/bed control unit 12 controls the filter switching mechanism 62 to interpose the X-ray filter 61a between the X-ray tube 5 and the object P when performing the first scan and to interpose the X-ray filter 61b between the X-ray tube 5 and the object P when performing the second scan.

The X-ray filter 61a is formed from a material having substantially the same atomic number as that a contrast material Xa to be administered to an object. The low tube voltage Va and the thickness ha of the X-ray filter 61a are set to values that make the object P be irradiated with X-rays whose spectrum is mostly distributed to an energy region lower than the K-absorption edge of the material Xa when the low tube voltage Va is applied to the X-ray tube 5. Note that the term "mostly" in this case means that, for example, the ratio of the area of a spectrum in an energy region lower than the K-absorption edge of the material Xa to the total area of the spectrum exceeds a predetermined ratio (e.g., about 90%).

An X-ray absorption coefficient rises sharply before and after the K-absorption edge of a given material. That is, with regard to the X-rays generated by the X-ray tube 5 upon application of the low tube voltage Va, it is difficult for an energy band portion higher than the K-absorption edge of the material Xa to be transmitted through the X-ray filter 61a, whereas it is easy for an energy band portion lower than the K-absorption edge to be transmitted through the X-ray filter 61a. With the use of these properties, an X-ray spectrum on the low energy side when performing dual energy scanning can be formed in an energy region lower than the material Xa of the contrast medium. The specific values of the low tube voltage Va and the thickness ha may be determined theoretically or experimentally so as to obtain a desired X-ray spectrum in an energy band lower than the K-absorption edge of the material Xa.

The X-ray filter 61b is formed from a material different from the contrast material Xa and the X-ray filter 61a. Typically, the X-ray filter 61b is formed from a material having a higher atomic number than the contrast material Xa and the X-ray filter 61a. However, this description does not deny the possibility that the second filter 61b may be formed from a material having a lower atomic number than the contrast material Xa and the filter 61a.

The low tube voltage Vb and the thickness hb of the X-ray filter 61b are set to values that make the object P be irradiated with X-rays whose spectrum is mostly distributed to an energy region higher than the K-absorption edge of the material Xa when the high tube voltage Vb is applied to the X-ray tube 5. Note that the term "mostly" in this case means that, for example, the ratio of the area of a spectrum in an energy region higher than the K-absorption edge to the total area of the spectrum exceeds a predetermined ratio (e.g., about 90%). The specific values of the high tube voltage Vb and the thickness hb may be determined theoretically or experimentally so as to obtain a desired X-ray spectrum in an energy band higher than the K-absorption edge of the material Xb.

Two examples concerning the tube voltages Va and Vb, the materials Xa and Xb, and the thicknesses ha and hb will be described.

### [Specific Example 1]

Specific Example 1 will be described, in which the low tube voltage Va is set to 40 kV, the high tube voltage Vb is set to 50 kV, the material Xa is formed from iodine, the material Xb is formed from indium and zinc, the thickness ha is set to 250 µm, and the thickness hb is set to 300 µm (indium: 150 µm + zinc: 150 µm).

FIG. 4 is a graph showing an absorption coefficient CI of iodine, a spectrum Sa of X-rays emerging from the X-ray filter 61a when the low tube voltage Va is applied to the X-ray tube 5, and a spectrum Sb of X-rays emerging from the X-ray filter 61b when the high tube voltage Vb is applied to the X-ray tube 5. The abscissa represents energy (keV), the left ordinate represents normalized photon count, and the right ordinate represents absorption coefficient (1/cm).

The K-absorption edge of iodine appears at about 33 keV. The absorption coefficient CI changes by an order of magnitude before and after the K-absorption edge. Therefore, the X-rays generated from the X-ray tube 5 upon application of a voltage of 40 kV as the low tube voltage Va to the X-ray tube 5 are absorbed by the X-ray filter 61a more efficiently in the region of about 33 keV to 40 kV than in the remaining regions. As a result, the spectrum Sa is shaped so as to be mostly distributed to an energy region (about 15 keV to 33 keV) lower than the K-absorption edge of iodine.

On the other hand, as is obvious from FIG. 4, the spectrum Sb is mostly distributed to an energy region (about 33 keV to 50 keV) higher than the K-absorption edge of iodine under the condition of the high tube voltage Vb, the material Xb, and the thickness hb in this case.

The high voltage generation unit 10 generates the low tube voltage Va. At this time, the filter switching mechanism 62 arranges the X-ray filter 61a on an X-ray beam. As described above, the filter 61a has properties exhibiting high transparency with respect to a low-energy band and low transparency with respect to a high-energy band. Therefore, the spectrum Sa at this time is focused on a low energy band less than the K-absorption edge. On the other hand, when the high voltage generation unit 10 generates the high tube voltage Vb, the filter switching mechanism 62 arranges the filter 61b on an X-ray beam. The filter 61b has properties exhibiting high transparency with respect to a high-energy band and low transparency with respect to a low-energy band. Therefore, the spectrum Sb at this time is focused on a high energy band less than the K-absorption edge. This makes it possible to minimize the crosstalk between the spectrum Sa and the spectrum Sb.

### [Specific Example 2]

Specific Example 2 will be described, in which the low tube voltage Va is set to 60 kV, the high tube voltage Vb is set to 70 kV, the material Xa is formed from gadolinium, the material Xb is formed from indium and lead, the thickness ha is set to 300 µm, and the thickness hb is set to 800 µm (indium: 500 µm + lead: 300 µm).

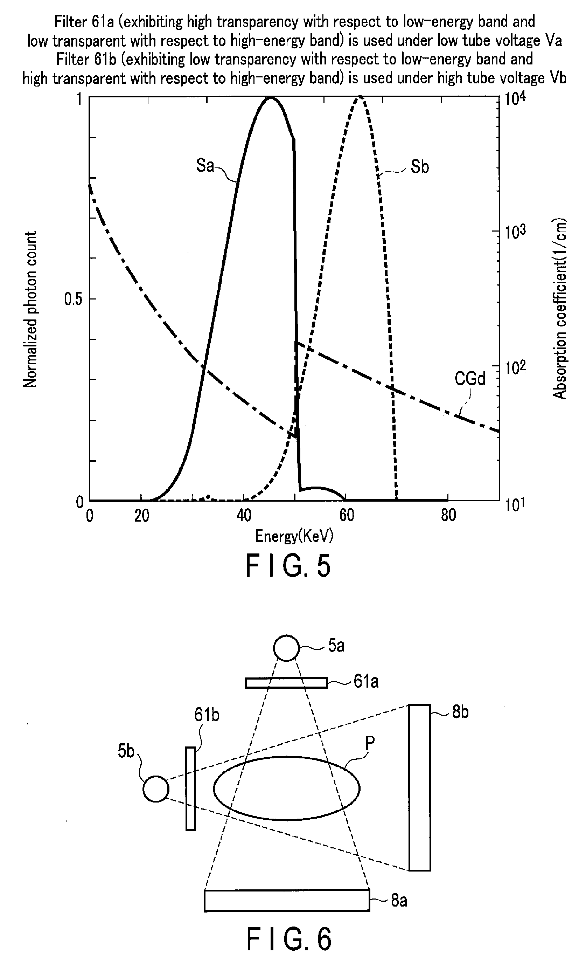

FIG. 5 is a graph showing an absorption coefficient CGd of gadolinium, the spectrum Sa of X-rays emerging from the X-ray filter 61a when the low tube voltage Va is applied to the X-ray tube 5, and the spectrum Sb of X-rays emerging from the X-ray filter 61b when the high tube voltage Vb is applied to the X-ray tube 5. The abscissa represents energy (keV), the left ordinate represents normalized photon count, and the right ordinate represents absorption coefficient (1/cm).

The K-absorption edge of gadolinium appears at about 50 keV. The absorption coefficient CGd changes by an order of magnitude before and after the K-absorption edge. Therefore, the X-rays generated from the X-ray tube 5 upon application of a voltage of 60 kV as the low tube voltage Va to the X-ray tube 5 are absorbed by the X-ray filter 61a more efficiently in the region of about 50 keV to 60 kV than in the remaining regions. As a result, the spectrum Sa is shaped so as to be mostly distributed to an energy region (about 25 keV to 50 keV) lower than the K-absorption edge of gadolinium.

On the other hand, as is obvious from FIG. 5, the spectrum Sb is mostly distributed to an energy region (about 50 keV to 70 keV) higher than the K-absorption edge of gadolinium under the condition of the high tube voltage Vb, the material Xb, and the thickness hb in this example.

As in this example, it is possible to minimize the crosstalk between the spectrum Sa and the spectrum Sb.

In the two examples described above as well, it is possible to irradiate the object P with X-rays having a spectrum mostly distributed to an energy region lower than the K-absorption edge of the material Xa forming the X-ray filter 61a and X-rays having a spectrum mostly distributed to an energy region higher than the K-absorption edge. As is obvious from FIGS. 4 and 5, the energy crosstalk in such X-rays is greatly reduced.

X-rays having energy lower than the K-absorption edge of a contrast medium material easily reach the X-ray detector 8 without being absorbed by the contrast medium. In contrast to this, X-rays having energy higher than the K-absorption edge of the contrast medium material are efficiently absorbed by the contrast medium and hence do not easily reach the X-ray detector 8. It is therefore possible to obtain a high-contrast X-ray CT image, with the contrast-enhanced area of the object P being clearly depicted, by making the image processing unit 44 perform weighted addition, at a predetermined ratio, with respect to the image obtained by reconstructing the projection data acquired by using, for example, these two types of X-rays.

In addition, when obtaining an X-ray CT image with focus being placed on a specific material in this manner, since X-rays corresponding to energy crosstalk having no contribution to substance separation are reduced, it is possible to suppress the exposure dose of the object P to a low level.

In addition to them, various preferable effects can be obtained from the arrangement disclosed in this embodiment. Using the X-ray filters and tube voltage control according to this embodiment allows even a current CT apparatus without the X-ray filters and tube voltage control to easily perform K-edge imaging.

### (Modification)

Several modifications will be described.

The above embodiment has exemplified the case in which the material Xa forming the X-ray filter 61a is formed from iodine and gadolinium. However, the material Xa may be properly selected in accordance with a target material such as a contrast medium or a specific tissue of the object P. In addition, it is not always necessary to match the material Xa with a target material. For example, if the atomic number of a target material is Z0, the material Xa may be a material corresponding to an atomic number Z given by Z = Z0 + 1 or Z = Z0 + 2. The K-absorption edge of the material shifts in the high-energy direction with an increase in atomic number. Even if, therefore, the material Xa is a material corresponding to an atomic number slightly shifting from the atomic number of the target material, it is possible to obtain an image capturing a target material with relatively high contrast.

The above embodiment has exemplified the X-ray CT apparatus 1 which switches between the low tube voltage Va and the high tube voltage Vb to be applied to the X-ray tube 5 every time the X-ray tube 5 makes one rotation. However, the technical idea disclosed in the above embodiment can also be applied to, for example, the dual-tube CT system, the fast switching CT system, and the dual-layer CT system which have already been described above.

The dual-tube CT system to which the technical idea is applied will be described as an example with reference to FIG. 6.

This dual-tube CT system includes two X-ray tubes 5a and 5b and two X-ray detectors 8a and 8b. The X-ray tube 5a and the X-ray detector 8a are provided on the rotating portion of the gantry device so as to face each other. Likewise, the X-ray tube 5b and the X-ray detector 8b are provided on the rotating portion of the gantry device so as to face each other. The X-ray tube 5a generates X-rays having a spectrum distributed to a low energy region upon reception of the low tube voltage Va. The X-ray detector 8a detects the X-rays generated by the X-ray tube 5a and transmitted through the object P. The X-ray tube 5b generates X-rays having a spectrum distributed to a high energy region upon reception of the high tube voltage Vb. The X-ray detector 8b detects the X-rays generated by the X-ray tube 5b and transmitted through the object P.

In the dual-tube CT system, the X-ray filter 61a is provided between the X-ray tube 5a and the object P, and the X-ray filter 61b is provided between the X-ray tube 5b and the object P. The X-ray filter 61a is attached to a wedge filter for adjusting the radiation quality of X-rays generated by, for example, the X-ray tube 5a. The X-ray filter 61b is attached to a wedge filter for adjusting the radiation quality of X-rays generated by, for example, the X-ray tube 5b. The materials Xa and Xb forming the X-ray filters 61a and 61b and the thicknesses ha and hb are the same as those described in the above embodiment. When executing scanning, this system acquires projection data while causing the X-ray tubes 5a and 5b to simultaneously generate X-rays, i.e., simultaneously executing the first and second scans.

Even when such a dual-tube CT system is constructed, the same effects as those in the above embodiment can be obtained.

There has been described the technique of switching between the two types of tube voltage Va and Vb and the two types of X-ray filters 61a and 61b having different radiation quality adjustment characteristics. As shown in FIG. 10, however, even if the two different tube voltages Va and Vb and the use and nonuse of the single type of X-ray filter 61a (or 61b) are switched, it is possible to improve the contrast of a contrast material.

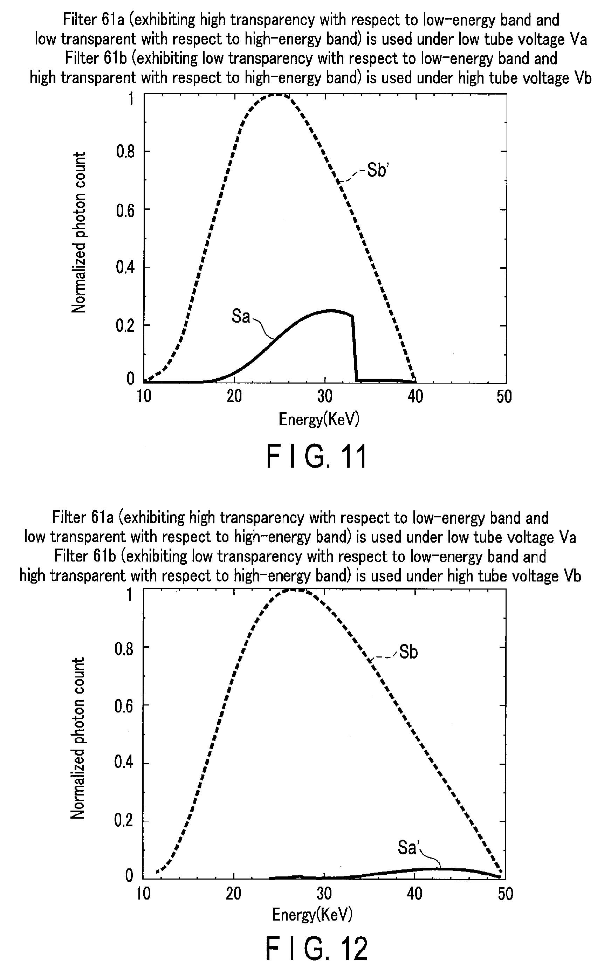

FIG. 11 shows spectra obtained when only the filter 61a is used. FIG. 11 shows a spectrum Sa obtained when the low tube voltage Va is selected and the filter 61a is interposed on an X-ray beam, and a spectrum Sb' obtained when the high tube voltage Vb is used while the filter 61a is removed from an X-ray beam and hence the filter 61b is not used. Even if the spectrum Sa is included by the spectrum Sb', it is possible to improve the contrast of a contrast material by specifying the differences in X-ray absorption coefficient between substances constituting an object based on the ratio between the spectra.

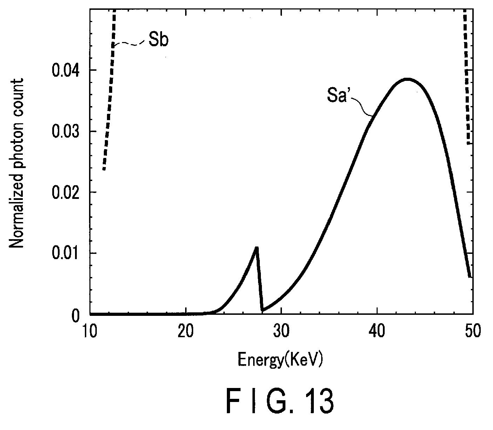

FIG. 12 shows a spectrum obtained when only the filter 61b is used. FIG. 13 shows the spectrum Sa' in FIG. 12 upon changing the scale. FIG. 13 shows the spectrum Sa' obtained when the low tube voltage Va is selected while the filter 61b is removed from an X-ray beam and hence the filter 61a is not used, and the spectrum Sb obtained when the high tube voltage Vb is used and the filter 61b is interposed on an X-ray beam. Even if the spectrum Sa' is included by the spectrum Sb, it is possible to improve the contrast of a contrast material by specifying the differences in X-ray absorption coefficient between substances constituting an object based on the ratio between the spectra.

Some embodiments of the present invention have been described above. However, these embodiments are presented merely as examples and are not intended to restrict the scope of the invention. These novel embodiments can be carried out in various other forms, and various omissions, replacements, and alterations can be made without departing from the spirit of the invention. The embodiments and their modifications are also incorporated in the scope and the spirit of the invention as well as in the invention described in the claims and their equivalents.

### Reference Signs List

- 1: X-ray CT apparatus
- 2: gantry device
- 3: bed device
- 4: console device
- 5: X-ray tube
- 6: filter unit
- 8: X-ray detector
- 12: gantry/bed control unit
- 13: data acquisition unit
- 60a, 60b: wedge filter
- 611, 61b: X-ray filter
- 62: filter switching mechanism
- Sa: X-ray spectrum (low tube voltage)
- Sb: X-ray spectrum (high tube voltage)
- CI: X-ray absorption coefficient (iodine)
- CGd: X-ray absorption coefficient (gadolinium)

## Claims

1. An X-ray CT apparatus comprising:
an X-ray tube;
a high voltage generation unit configured to selectively generate a first tube voltage to be applied to the X-ray tube and a second tube voltage higher than the first tube voltage;
a first filter formed from a material having substantially the same atomic number as that of a contrast material to be administered to an object and configured to perform radiation quality adjustment;
a second filter formed from a material different from the contrast material and configured to perform radiation quality adjustment;
a filter switching mechanism configured to switch between the first filter and the second filter to be interposed between the X-ray tube and the object;
an X-ray detector configured to detect X-rays transmitted through the object;
a reconstruction unit configured to reconstruct an image based on projection data obtained by an output from the X-ray detector; and
a control unit configured to control the high voltage generation unit and the filter switching mechanism to synchronize switching between the first tube voltage and the second tube voltage with switching between the first filter and the second filter.

2. The X-ray CT apparatus of claim 1, wherein the first filter is arranged between the X-ray tube and the object when the first tube voltage is selected, and the second filter is arranged between the X-ray tube and the object when the second tube voltage is selected.

3. The X-ray CT apparatus of claim 2, further comprising an image generation unit configured to generate a desired image from an image reconstructed based on the projection data acquired while the first tube voltage is selected and the first filter is arranged and an image reconstructed based on the projection data acquired while the second tube voltage is selected and the second filter is arranged.

4. The X-ray CT apparatus of claim 3, wherein the first tube voltage is set such that more than 90% of a spectrum of X-rays passing through the first filter are distributed to an energy band lower than a K-absorption edge of the contrast material.

5. The X-ray CT apparatus of claim 4, wherein the second tube voltage is set such that more than 90% of a spectrum of X-rays passing through the second filter are distributed to an energy band higher than the K-absorption edge of the contrast material.

6. The X-ray CT apparatus of claim 1, wherein the control unit alternately switches between a state in which the first tube voltage is selected and the first filter is arranged and a state in which the second tube voltage is selected and the second filter is arranged, every time the X-ray tube rotates through an angle range necessary for reconstruction of the image.

7. The X-ray CT apparatus of claim 1, wherein the control unit alternately switches between a state in which the first tube voltage is selected and the first filter is arranged and a state in which the second tube voltage is selected and the second filter is arranged, every time the X-ray tube rotates through a view pitch or an integer multiple thereof.

8. The X-ray CT apparatus of claim 1, wherein the first filter and the second filter each are provided on a wedge filter.

9. An X-ray CT apparatus comprising:
an X-ray tube;
a high voltage generation unit configured to selectively generate a first tube voltage to be applied to the X-ray tube and a second tube voltage higher than the first tube voltage;
a filter formed from a material having substantially the same atomic number as that of a contrast material to be administered to an object and configured to perform radiation quality adjustment;
a filter insertion/removal mechanism configured to insert/remove the filter between the X-ray tube and the object;
an X-ray detector configured to detect X-rays transmitted through the object;
a reconstruction unit configured to reconstruct an image based on an output from the X-ray detector; and
a control unit configured to control the high voltage generation unit and the filter insertion/removal mechanism to synchronize switching between the first tube voltage and the second tube voltage with insertion/removal of the filter.

10. The X-ray CT apparatus of claim 9, wherein the filter is arranged between the X-ray tube and the object when the first tube voltage is selected, and the filter is removed from between the X-ray tube and the object when the second tube voltage is selected.

11. The X-ray CT apparatus of claim 10, further comprising an image generation unit configured to generate a desired image from an image reconstructed based on the projection data acquired while the first tube voltage is selected and the filter is arranged and an image reconstructed based on the projection data acquired while the second tube voltage is selected and the filter is removed.

12. The X-ray CT apparatus of claim 9, wherein the control unit alternately switches between a state in which the first tube voltage is selected and the filter is arranged and a state in which the second tube voltage is selected and the filter is removed, every time the X-ray tube rotates through an angle range necessary for reconstruction of the image.

13. The X-ray CT apparatus of claim 9, wherein the control unit alternately switches between a state in which the first tube voltage is selected and the filter is arranged and a state in which the second tube voltage is selected and the filter is removed, every time the X-ray tube rotates through a view pitch or an integer multiple thereof.

14. An X-ray CT apparatus An X-ray CT apparatus comprising:
an X-ray tube;
a high voltage generation unit configured to selectively generate a first tube voltage to be applied to the X-ray tube and a second tube voltage higher than the first tube voltage;
a filter formed from a material different from a contrast material to be administered to an object and configured to perform radiation quality adjustment;
a filter insertion/removal mechanism configured to insert/remove the filter between the X-ray tube and the object;
an X-ray detector configured to detect X-rays transmitted through the object;
a reconstruction unit configured to reconstruct an image based on an output from the X-ray detector; and
a control unit configured to control the high voltage generation unit and the filter insertion/removal mechanism to synchronize switching between the first tube voltage and the second tube voltage with insertion/removal of the filter.

15. The X-ray CT apparatus of claim 14, wherein the filter is removed from between the X-ray tube and the object when the first tube voltage is selected, and the filter is arranged between the X-ray tube and the object when the second tube voltage is selected.

16. The X-ray CT apparatus of claim 15, further comprising an image generation unit configured to generate a desired image from an image reconstructed based on the projection data acquired while the first tube voltage is selected and the filter is removed and an image reconstructed based on the projection data acquired while the second tube voltage is selected and the filter is arranged.

17. The X-ray CT apparatus of claim 14, wherein the control unit alternately switches between a state in which the first tube voltage is selected and the filter is removed and a state in which the second tube voltage is selected and the filter is arranged, every time the X-ray tube rotates through an angle range necessary for reconstruction of the image.

18. The X-ray CT apparatus of claim 14, wherein the control unit alternately switches between a state in which the first tube voltage is selected and the filter is removed and a state in which the second tube voltage is selected and the filter is arranged, every time the X-ray tube rotates through a view pitch or an integer multiple thereof.
